(19) Europäisches Patentamt
European Patent Office
Office européen des brevets

(11) Veröffentlichungsnummer: **0 093 444**
**B1**

# (12) EUROPÄISCHE PATENTSCHRIFT

(45) Veröffentlichungstag der Patentschrift: **16.05.90**

(51) Int. Cl.[5]: **C 07 D 263/56,** C 07 D 235/20, C 07 D 277/66

(21) Anmeldenummer: **83104265.0**

(22) Anmeldetag: **30.04.83**

## (54) Verfahren zur Herstellung von 4,4'-Bis-benz-ox(-thi,-imid)-azol-2-yl-stilbenen.

(30) Priorität: **05.05.82 DE 3216723**

(43) Veröffentlichungstag der Anmeldung: **09.11.83 Patentblatt 83/45**

(45) Bekanntmachung des Hinweises auf die Patenterteilung: **16.05.90 Patentblatt 90/20**

(84) Benannte Vertragsstaaten: **DE**

(56) Entgegenhaltungen:
**US-A-3 586 673**

**Chemical Abstracts Band 83, Nr. 12, 22 September 1975, Columbus, Ohio, USA; Seite 93, Spalte 2, Abstract Nr. 99224z KHIMIYA GETEROTSIKLICHESKIKH SOEDINENII, Band 1981E.M. VERNIGOR et al. "Synthesis and spectral-luminescence properties of azole analogs of 1,4-distyrylbenzene and isomeric distyrylnaphthalenes", Seiten 464-468**

(73) Patentinhaber: **HOECHST AKTIENGESELLSCHAFT**
**Postfach 80 03 20**
**D-6230 Frankfurt am Main 80 (DE)**

(72) Erfinder: **Heiss, Lorenz, Dr.**
**Stormstrasse 39**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 093 444 B1

**Beschreibung**

Aus Khim. Geterotsikl. Soedin 1981, S. 463—467 ist bereits ein Verfahren zur Herstellung von Bisbenzoxazolyl-stilbenen bekannt, bei dem 2-Benzoxazolyl-(4-brommethyl)-benzol mit Kalium-tertiär-butylat dehydrobromiert wird. Nachteilig bei diesem Verfahren ist besonders die Tatsache, daß dabei ein ca. vier- bis achtfacher Überschuß des teuren Kalium-tertiär-Butylats erforderlich ist.

Es wurde nun ein verbessertes Verfahren zur Herstellung von 4,4-Bis-benz-ox(-thi, imid)-azol-2-yl-stilbenen der Formel

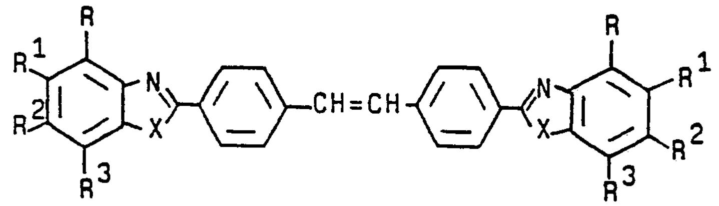

worin R, $R^1$, $R^2$ und $R^3$ gleich oder verschieden sind und Wasserstoff, $C_1$—$C_4$-Alkyl, $C_1$—$C_4$-Alkoxy, $C_1$—$C_4$-Alkoxycarbonyl, Chlor, Brom, Nitro oder Sulfo und X Sauerstoff, Schwefel oder NH bedeuten, durch Dehydrohalogenierung von 2-Benz-ox-(-thi, -imid)-azolyl-4-halomethylbenzolen der Formel

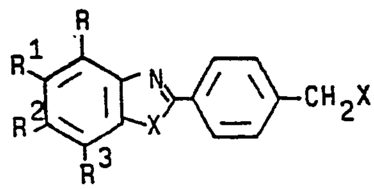

wobei X Cl oder Br bedeutet und R, $R^1$, $R^2$ und $R^3$ die oben angegebenen Bedeutungen haben, in einem aprotischen polaren Lösemittel und in Gegenwart einer starken Base gefunden. Dieses Verfahren besteht darin, daß man als starke Base eine etwa äquivalente Menge an Natrium-tert.-butylat nimmt methylbenzole mit einer etwa equivalenten Menge Natrium-tert.-butylat reagieren läßt.

Die als Ausgangsverbindungen dienenden (4-Halomethyl)-iminobenzoesäurealkylester werden hergestellt, indem man p-Cyanbenzylchlorid oder p-Cyanbenzylbromid in einem Überschuß des Alkohols der Formel R—OH dispergiert und Chlor- oder Bromwasserstoff einleitet. Man erhält so die HCl oder HBr-Salze der Imino-ester der oben angegebenen Formel.

Diese Salze werden dann mit einem o-Aminophenol, o-Aminothiophenol oder o-Phenylendiamin umgesetzt und zwar durch Erhitzen in einem inerten organischen Lösemittel, vorzugsweise in einem niederen Alkohol, bei Temperaturen von Raumtemperatur bis zur Siedetemperatur des Lösemittels.

Es wurde gefunden, daß man die Ausbeuten dieser Reaktion erheblich erhöhen kann, wenn man im Überschuß von schwachen wasserfreien Säuren, vorzugsweise Essigsäure, arbeitet, um die Nebenreaktion, eine Alkylierung durch die Chlormethylgruppe auszuschließen. Dies ist überraschend, da man bei Überschuß einer starken Säure eine drastische Minderung der Ausbeuten erhält.

Es ist auch möglich, 2-Benzoxazolyl-4-halomethylbenzol direkt aus p-Cyanbenzylchlorid oder p-Cyanbenzylbromid ohne Zwischenisolierung der Iminoester auf folgende Weise herzustellen. Man dispergiert zunächst p-Cyanbenzylchlorid oder p-Cyanbenzylbromid in einem Überschuß des Alkohols der Formel R—OH, leitet Chlor- bzw. Bromwasserstoff ein und läßt einige Stunden ausreagieren bis im Infrarot-Spektrum die Nitrilbande bei 2220 $cm^{-1}$ verschwunden ist. Der Überschuß an Halogenwasserstoff wird dann mit einer Base, vorzugsweise mit Soda neutralisiert und das Reaktionsgemisch wird erneut durch Zugabe einer schwachen Säure, vorzugsweise mit Essigsäure angesäuert. Anschließend gibt man o-Aminophenol, o-Aminothiophenol oder o-Phenylendiamin zu und erhitzt auf Temperaturen von 30 bis 80°C.

Nach Beendigung der Reaktion gibt man Wasser zu, saugt das Reaktionsprodukt ab und wäscht mit Wasser nach. Man erhält so das 2-Benzoxazolyl-4-halomethylbenzol bzw. die entsprechenden Benzthiazolyl- oder Benzimidazolyl-Verbindungen.

Zur Herstellung der Stilben-Verbindungen werden diese Halomethylbenzol-Verbindungen in einem aprotischen polaren Lösemittel gelöst und durch Zugabe einer ungefähr equivalenten Menge an Natrium-tert.-butylat dehydrohalogeniert. Bevorzugtes Lösemittel ist Dimethylformamid. Die Reaktionstemperatur liegt bei ca. 10 bis 50°C. Vorzugsweise arbeitet man so, daß man zunächst in situ das Natrium-tert.-butylat in dem Lösemittel erzeugt aus Natriumhydrid und tert.-Butanol. Zu dieser Dispersion gibt man dann die Halomethylbenzol-Verbindung. Zur Aufarbeitung gibt man zu dem Reaktionsgemisch zunächst Wasser zu und stellt mit Mineralsäure schwach sauer. Die Stilbenverbindung wird dann wie üblich abfiltriert, gewaschen und getrocknet. Man erhält diese Stilbenverbindungen analysenrein in quantitativer Ausbeute.

Der Vorteil dieser Dehydrohalogenierung in Dimethylformamid mit Natrium-tert.-butylat gegenüber dem Verfahren wie es in Khim. Geterotsikl. Soedin 1981, S. 463—467 beschrieben ist, liegt darin, daß hier überraschenderweise eine quantitative Ausbeute erreicht wird mit der equivalenten Menge an Natrium-tert.-butylat, während bei dem vorbekannten Verfahren die Ausbeuten geringer sind trotz Einsatzes eines vier- bis achtfachen Überschusses an Kalium-tert.-Butylat. Außerdem war es überraschend, daß die Synthese in Dimethylformamid wesentlich reinere Produkte liefert als in Dimethylsulfoxid sowie die Tatsache, daß sich die Halomethylbenzole in Dimethylformamid wesentlich besser lösen als in Dimethylsulfoxid. Dadurch ergibt sich eine Verbesserung der Raum-Zeit-Ausbeute.

Die Stilbenverbindungen der Formel 1 werden als optische Aufheller verwendet.

## Beispiel

110 g (0,5 Mol) p-Chlormethyl-iminobenzoesäure-methylester-Hydrochlorid und 54 g (0,5 Mol) o-Aminophenol werden in 800 ml Methanol gelöst und etwa 1 Stunde bei 65°C gerührt. Man kühlt dann auf Raumtemperatur ab, gibt zur Lösung des gebildeten Ammoniumchlorids etwa 500 ml Wasser zu und saugt ab. Man wäscht mit Wasser nach und erhält nach dem Trocknen 110 g Benzoxazolyl-2-(4-chlormethyl)-benzol (90,5% d.Th.). Schmelzpunkt: 150—151°C.

gefunden: C = 69,0% H = 4,1% N = 5,7% Cl = 14,5%
berechnet: C = 69,1% H = 4,1% N = 5,8% Cl = 14,6%

In 150 ml Dimethylformamid werden unter Stickstoffatmosphäre 3,6 g Tertiärbutanol und 1,6 g Natriumhydrid (80% ig) dispergiert und eine Stunde gerührt, dan fügt man unter Kühlung bei 20°C, 9,8 g (0,04 M) Benzoxazolyl-2-(4-chlormethyl)-benzol hinzu und rührt sechs Studen bei 20°C. Mit 100 ml Wasser verdünnt man und stellt mit Salzsäure auf pH 5—6. Nach dem Absaugen wird mit Wasser und Methanol gründlich nachgewaschen und getrocknet. Man erhält in grünlichen Kristallen 4,4'-Bisbenzoxazol-2-yl-stilben in einer Ausbeute von 8,1 g (97,8%). Schmelzpunkt: 355—360°C.

gefunden: C 80,6% H 4,3% N 6,8%
berechnet: C 81,1% H 4,3% N 6,8%

In gleicher Weise wurden zunächst die in der folgenden Tabelle aufgeführten 4-Halomethylbenzole hergestellt, aus denen dann die in Tabelle 2 aufgeführten Stilbenverbindungen gewonnen wurden.

**Tabelle 1**

| Beisp. | Ausgangsprodukte | Endprodukte | Schmelzpkt. | C, H, Cl-Analyse | Ausbeute |
|---|---|---|---|---|---|
| 2) | 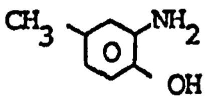 | 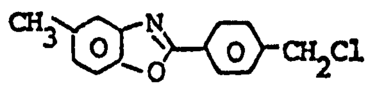 | 144°C | gef. 69,7%; 4,8%; 13,5 %<br>ber. 69,9%; 4,7%; 13,8 % | 91 % |
| 3) | 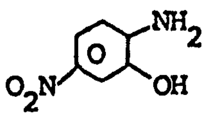 | 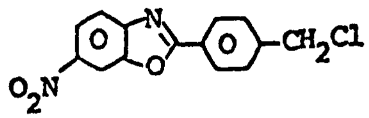 | 138°C | gef. 58,1%; 3,3%; 11,9 %<br>ber. 58,2%; 3,1%; 12,2 % | 88 % |
| 4) | 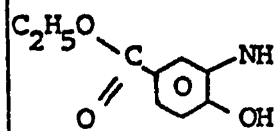 | 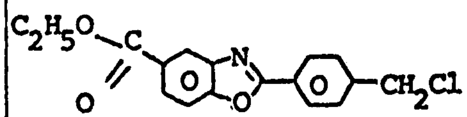 | 137°C | gef. 64,6%; 4,4%; 11,0 %<br>ber. 64,8%; 4,4%; 11,3 % | 82 % |
| 5) | 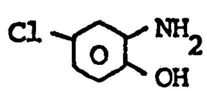 | 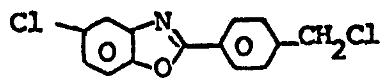 | 167°C | gef. 60,0%; 3,3%; 24,9 %<br>ber. 60,3%; 3,2%; 25,5 % | 86 % |
| 6) | 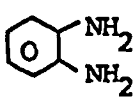 | 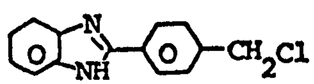 | subl.<br>200°C | gef. 68,9%; 4,8%; 14,2 %<br>ber. 69,2%; 4,6%; 14,6 % | 90 % |
| 7) | 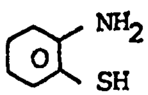 | 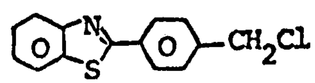 | 114°C | gef. 64,3%; 3,8%; 13,5 %<br>ber. 64,9%; 3,9%; 13,7 % | 56 % |
| 8) | 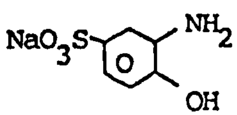 | 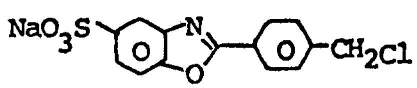 | - | gef. 48,3%; 2,7%; 10,2 %<br>ber. 48,7%; 2,6%; 10,2 % | 51 % |

Tabelle 2

| Beisp. | Ausgangsprodukte | Endprodukte | Schmelzpkt. | C, H, N-Analyse | Ausbeute |
|---|---|---|---|---|---|
| 2) | 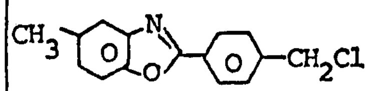 | 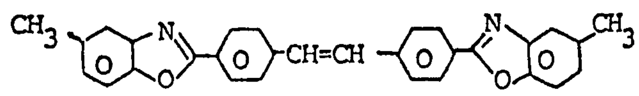 | 280-285°C | gef.80,8%;5,1%;6,2%<br>ber.81,2%;5,2%;6,3% | 94 % |
| 3) | 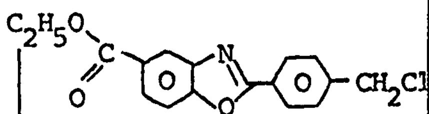 | 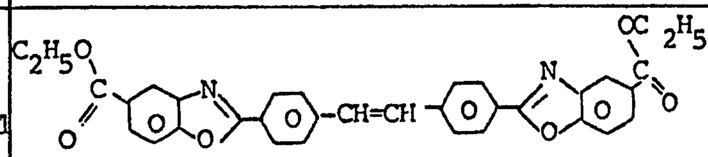 | 272-278°C | gef.72,8%;4,8%;5,1%<br>ber.73,1%;4,7%;5,0% | 88 % |
| 4) | 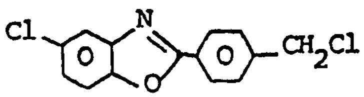 | 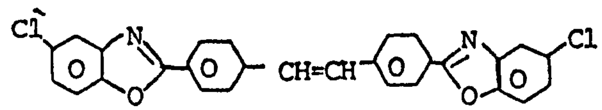 | 306-310°C | gef.69,1%;3,3%;5,7%<br>ber.69,6%;3,3%;5,8% | 91 % |
| 5) | 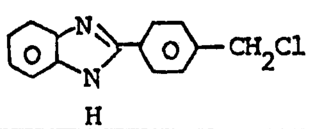 | 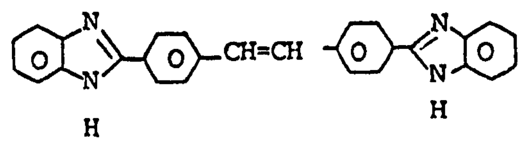 | >340°C Zers | gef.81,2%;4,7%;13,5%<br>ber.81,6%;4,8%;13,6% | 86 % |
| 6) | 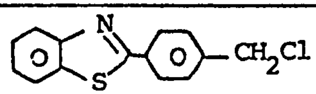 | 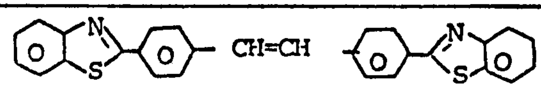 | 302-308°C | gef.75,0%;4,1%;3,1%<br>ber.75,3%;4,0%;3,1% | 72 % |
| 7) | 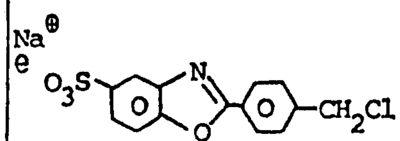 | 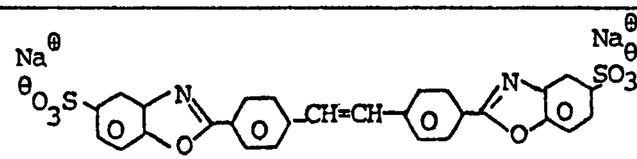 | >360°C | gef.53,9%;2,5%;4,4%<br>ber.54,4%;2,6%;4,5% | 76 % |
| 8) | 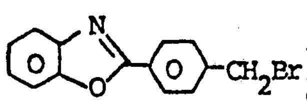 | 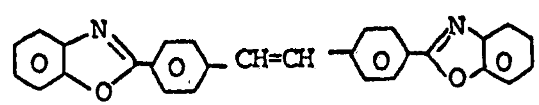 | 358-362°C | gef.80,7%;4,3%;6,7%<br>ber.81,1%;4,3%;6,8% | 90 % |

**Patentansprüche**

1. Verfahren zur Herstellung von 4,4-Bis-benz-ox(-thi, -imid)-azol-2-yl-stilbenen der Formel

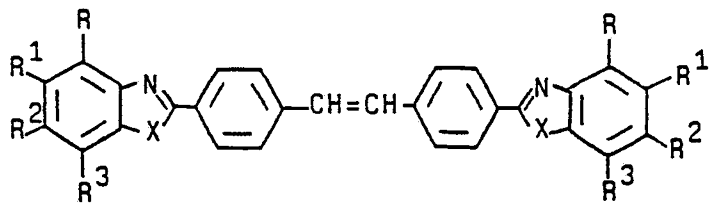

worin R, $R^1$, $R^2$ und $R^3$ gleich oder verschieden sind und Wasserstoff, $C_1$—$C_4$-Alkyl, $C_1$—$C_4$-Alkoxy, $C_1$—$C_4$-Alkoxycarbonyl, Chlor, Brom, Nitro oder Sulfo und X Sauerstoff, Schwefel oder NH bedeuten, durch Dehydrohalogenierung von 2-Benz-ox(-thi, -imid)-azolyl-4-halomethylbenzolen der Formel

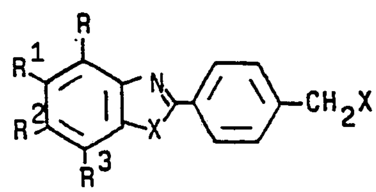

wobei X Cl oder Br bedeutet und R, $R^1$, $R^2$ und $R^3$ die oben angegebenen Bedeutungen haben, in einem aprotischen polaren Lösemittel und in Gegenwart einer starken Base, dadurch gekennzeichnet, daß man als starke Base eine etwa äquimolare Menge an Natrium-tert.-butylat nimmt.

2. Verfahren nach Anspruch 1, dadurch gekennzeichnet, daß man die Reaktion in Dimethylformamid durchführt.

3. Verfahren nach Anspruch 1, dadurch gekennzeichnet, daß man Natrium-tert.-butylat in situ aus Natriumhydrid und Butanol erzeugt.

**Revendications**

1. Procédé pour préparer des bis-(benzoxazolyl-2 ou benzothiazolyl-2 ou benzimidazolyl-2)-4,4' stilbènes qui répondent à la formule:

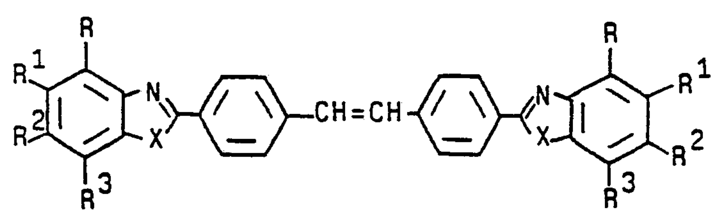

dans laquelle R, $R^1$, $R^2$ et $R^3$ représentent chacun, indépendamment les uns des autres, l'hydrogène, un alkyle en $C_1$—$C_4$, un alcoxy en $C_1$—$C_4$, un alcoxycarbonyle en $C_1$—$C_4$, le chlore, le brome, un nitro ou un radical sulfo, et X représente l'oxygène, le soufre ou un radical NH, par déshydrohalogénation de (benzoxazolyl-2 ou benzothiazolyl-2 ou benzimidazolyl-2)-1 halogénométhyl-4 benzènes répondant à la formule:

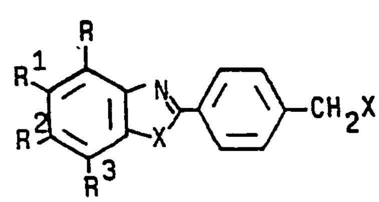

dans laquelle X représente Cl ou Br, et R, $R^1$, $R^2$ et $R^3$ ont la signification qui leur ont été données ci-dessus, dans un solvent polaire aprotique et en présence d'une base forte, procédé caractérisé en ce qu'on utilise, comme base forte, un quantité à peu près équimolaire de tert-butylate de sodium.

2. Procédé selon la revendication 1 caractérisé en ce qu'on effectue la réaction dans du diméthylformamide.

3. Procédé selon la revendication 1 caractérisé en ce qu'on crée le tert-butylate de sodium in situ à partir d'hydrure de sodium et de butanol.

## Claims

1. A process of the preparation of a 4,4-bis-[benz(ox, othi or imid)azol-2-yl]stilbene of the formula

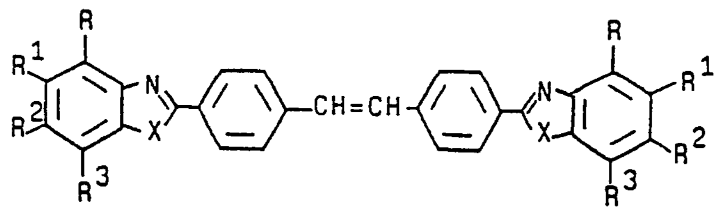

in which R, $R^1$, $R^2$ and $R^3$ are identical or different and denote hydrogen, $C_1$—$C_4$-alkyl, $C_1$—$C_4$-alkoxy, $C_1$—$C_4$-alkoxycarbonyl, chlorine, bromine, nitro or sulfo and X denotes oxygen, sulfur or NH, by dehydrohalogenating a 2-benz(ox, othi or imid)azolyl-4-halomethylbenzene of the formula

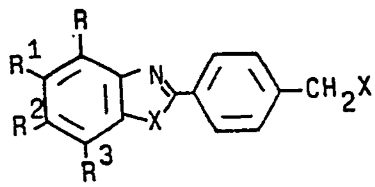

wherein X denotes Cl or Br, and R, $R^1$, $R^2$ and $R^3$ have the meanings indicated above, in an aprotic polar solvent and in the presence of a strong base, which comprises taking as strong base an approximately equimolar amount of sodium tert.-butylate.

2. The process as claimed in claim 1, wherein the reaction is carried out in dimethylformamide.

3. The process as claimed in claim 1, wherein the sodium tert.-butylate is produced in situ from sodium hydride and butanol.